# EUROPÄISCHE PATENTSCHRIFT

(11) **EP 1 675 641 B1**
(45) Veröffentlichungstag und Bekanntmachung des Hinweises auf die Patenterteilung: **05.03.2008**
(21) Anmeldenummer: 04764789.6
(22) Anmeldetag: 03.09.2004
(51) Int. Cl.: A61M 39/00

(54) **PORTSYSTEM**
ACCESS SYSTEM
SYSTEME D'ACCES

(30) Priorität: 02.10.2003 DE 10346470
(43) Veröffentlichungstag der Anmeldung: 05.07.2006
(73) Patentinhaber: Fresenius Kabi Deutschland GmbH, 61352 Bad Homburg v.d.H. (DE)
(72) Erfinder: HAINDL, Hans, 30974 Wennigsen (DE); JOCHUM, Christoph, 61130 Nidderau (DE); ROEDIGER, Frank, M., 30938 Thönse (DE)
(74) Vertreter: Luderschmidt, Schüler & Partner
(86) Internationale Anmeldenummer: PCT/EP2004/009834
(87) Internationale Veröffentlichungsnummer: WO 2005/032645

(56) Entgegenhaltungen:
- DE-A- 4 129 781
- US-A- 4 464 178
- US-A- 4 723 948
- US-A- 4 772 270
- US-A- 5 607 393
- US-A- 5 718 682
- US-B1- 6 213 973

## Beschreibung

Die Erfindung betrifft ein Portsystem, das implantiert wird, um einen Zugang zu einem entfernt liegenden Wirkort zu schaffen, an den ein Wirkstoff gelangen soll.

Das Portsystem weist ein unter die Haut implantierbares Gehäuse auf, in dem eine Kammer zur Aufnahme des Wirkstoffs angeordnet ist. Die Kammer des Ports wird von einer durchstechbaren Membran verschlossen, die unter der Haut liegt. Zum Zuspritzen des Wirkstoffs wird die Haut und die Membran mit einer Kanüle durchstochen und der Wirkstoff in die Kammer gespritzt. Von der Kammer gelangt der Wirkstoff dann über den Katheter zum Wirkort.

Die DE 41 29 782 C1 beschreibt einen Portsystem, das aus einem Port und einem Katheter besteht. Der Port weist ein Gehäuse mit einer unteren Ausnehmung zur Aufnahme des Wirkstoffs und einer oberen Ausnehmung zur Aufnahme der Membran auf. Die Membran wird in der Ausnehmung von einem Spannring gehalten, der einen Druck auf die Membran ausübt, so dass sich die Membran nach außen wölbt. Nachteilig ist, dass die Montage der Membran erschwert ist. Für eine Klebe- oder Schweißverbindung ist es erforderlich, den unter Druck stehenden Spannring zu fixieren. Wenn der Spannring beispielsweise verklebt werden soll, ist der Spannring so lange gegen das Gehäuse zu drücken, bis der Kleber ausgehärtet ist.

Zum Anschluss des Katheters weist der bekannte Port ein konisches Anschlussstück auf, das mit der zentralen Gehäuseausnehmung in Fluidverbindung steht. Auf das konische Anschlussstück wird der Katheter aufgeschoben. Der Katheter wird an dem Anschlussstück mittels einer Klemmhülse fixiert, die mit dem Gehäuse verschraubt wird. Eine derartige Schlauchkupplung ist in der DE 41 29 781 A1 im Einzelnen beschrieben. Nachteilig ist, dass nach dem Einsetzen der Klemmhülse nicht erkennbar ist, wie weit der Katheter auf das konische Anschlussstück aufgeschoben ist. Bei unsachgemäßer Montage besteht aber die Gefahr, dass sich der Schlauch von dem Anschlussstück löst. Nachteilig ist auch, dass die Klemmhülse als separates Teil leicht verloren gehen kann. Dadurch ist die Handhabung erschwert.

Bei der Injektion eines Wirkstoffes mit einer Kanüle ist darauf zu achten, dass die unter der Haut liegende Membran getroffen wird. Wenn die Kanüle aber nicht auf die Membran, sondern das Gehäuse trifft, besteht die Gefahr, dass die Nadel von dem Gehäuse abrutscht und den aus dem Gehäuse heraustretenden Katheter verletzt.

Die US 4,723,948A beschreibt einen Port mit einer Befestigungsvorrichtung für einen Katheter. Die Befestigungsvorrichtung verfügt über zwei Klemmbacken, die über ein Filmscharnier miteinander verbunden sind. In der geschlossenen Stellung umschließen die beiden Klemmbacken den Katheter, so dass der Katheter verklemmt ist. Der Sicherung der beiden Klemmbacken dient eine die Klemmbacken in der geschlossenen Position umschließende Hülse.

Der Erfindung liegt die Aufgabe zugrunde, einen Port für einen Katheter bereitzustellen, der sich ohne großen Montageaufwand einfach zusammensetzen lässt. Insbesondere ist die Aufgabe der Erfindung, einen Port zu schaffen, der eine einfache Fixierung des Katheters an dem Anschlussstück erlaubt.

Die Lösung dieser Aufgaben erfolgt erfindungsgemäß mit den Merkmalen des Patentanspruchs 1. Vorteilhafte Ausführungsformen sind Gegenstand der Unteransprüche.

Der erfindungsgemäße Port für einen Katheter weist zur Fixierung des Katheters an dem Anschlussstück zwei mit dem Gehäuse verbundene Klemmbacken mit einander gegenüberliegenden Klemmflächen auf. Die Klemmbacken sind aus einer ersten Position, in der sie von dem Gehäuse seitlich abstehen, in eine zweite Position bewegbar, in der sie den Katheter zwischen ihren Klemmflächen klemmend fixieren. Die von dem Gehäuse seitlich abstehenden Klemmbacken haben den Vorteil, dass sie beim Aufschieben des Katheters auf das Anschlussstück nicht die Sicht behindern. Somit kann der Sitz des Katheters auf dem Anschlussstück kontrolliert werden. Vorteilhaft ist auch, dass der Arzt beim Aufschieben des Katheters nicht von den Klemmbacken behindert wird. Zum Fixieren des Katheters an dem Anschlussstück brauchen die Klemmbacken lediglich in die zweite Position bewegt zu werden. Dadurch wird die Handhabung vereinfacht.

Bei einer bevorzugten Ausführungsform der Erfindung sind die Klemmbacken mit federnd ausgebildeten Befestigungsarmen an dem Gehäuse befestigt. Vorteilhaft ist, dass die Klemmbacken mit den federnden Befestigungsarmen einerseits sicher an dem Gehäuse befestigt, andererseits leicht zu bewegen sind. Vorteilhaft ist auch, dass die federnden Befestigungsarme keine aufwendigen Befestigungstechniken erforderlich machen. Grundsätzlich können die Klemmbacken aber auch mittels Gelenken oder Schiebern an dem Gehäuse befestigt sein.

Bei einer weiteren bevorzugten Ausführungsform bilden die Befestigungsarme eine das Gehäuse seitlich umfassende Spange, die an der dem Anschlussstück gegenüberliegenden Seite an dem Gehäuse befestigt ist. Dadurch wird der konstruktive Aufwand weiter vereinfacht. Die als Spange ausgebildeten Befestigungsarme können als separates Teil, beispielsweise als Spritzgussteil, hergestellt und später mit dem Gehäuse verbunden werden. Es ist aber auch möglich, dass die Spange zusammen mit dem Gehäuse hergestellt wird. Die Befestigung der Spange an der dem Anschlussstück gegenüberliegenden Seite des Gehäuses macht einen relativ großen Bewegungsbereich für die Klemmbacken möglich. Folglich können die Klemmbacken in der ersten Position relativ weit seitlich von dem Gehäuse abstehen, so dass ein möglichst großer Freiraum im Bereich des Anschlussstücks geschaffen wird.

Eine weitere bevorzugte Ausführungsform sieht vor, dass die Klemmbacken in der zweiten Position einrastend an dem Gehäuse festgelegt sind. Da die Klemmbacken an dem Gehäuse einen sicheren Halt finden, ist gewährleistet, dass sich die Verbindung nicht löst.

Das Gehäuse weist vorteilhafterweise seitliche Führungsnuten auf, in denen die Klemmbacken geführt sind. Dadurch wird sichergestellt, dass die Klemmbacken zwar seitlich abstehen können, dennoch einen ausreichenden Halt an dem Gehäuse finden.

Zur einrastenden Festlegung der Klemmbacken an dem Gehäuse sind vorteilhafterweise in den Führungsnuten Absätze ausgebildet, wobei die Klemmbacken Rasthaken aufweisen. Eine zusätzliche Fixierung kann dadurch erfolgen, dass die Klemmbacken einander zugeordnete Zapfen und Bohrungen aufweisen. Beim Zusammendrücken der Klemmbacken greifen die Zapfen in die Bohrungen, so dass eine formschlüssige Verbindung geschaffen wird.

Im Folgenden wird ein Ausführungsbeispiel der Erfindung unter Bezugnahme auf die Zeichnungen näher erläutert.

Es zeigen:
- Figur 1: ein Ausführungsbeispiel des Ports in der Draufsicht, wobei die Klemmbacken zur klemmenden Fixierung des an dem Port angeschlossenen Katheters an dem Gehäuse anliegen,
- Figur 2: den Port von Figur 1 in der Unteransicht,
- Figur 3: eine Explosionsdarstellung des Ports mit von dem Gehäuse abstehenden Klemmbacken in der Unteransicht,
- Figur 4: eine Unteransicht des Ports in einer Explosionsdarstellung mit abstehenden Klemmbacken aus einer anderen Richtung,
- Figur 5: den Port mit abstehenden Klemmbacken in der Draufsicht und
- Figur 6: einen Schnitt durch das Einsatzstück mit der Kammer zur Aufnahme des Wirkstoffes.

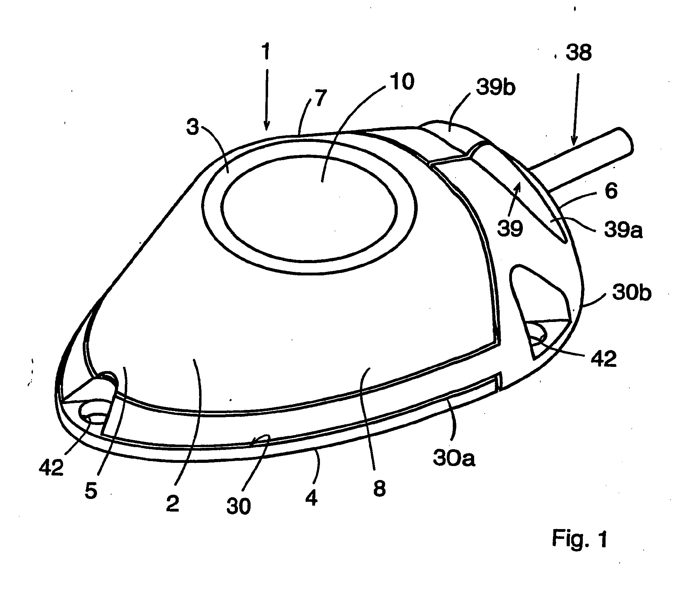

Figur 1 zeigt in vergrößerter Darstellung eine Draufsicht auf den Portkatheter, der einen Port 1 und einen Katheter 38 umfasst. Der Port 1, der etwa die Größe einer Fingerkuppe haben kann, weist ein flaches Gehäuse 2 auf, das unter die Haut implantiert wird. Die unter der Haut liegende Oberseite des Gehäuses 1 ist mit dem Bezugszeichen 3, die Unterseite des Gehäuses mit 4, die Vorderseite des Gehäuses mit 5, die Rückseite des Gehäuses mit 6 und die Längsseiten des Gehäuses mit 7 und 8 bezeichnet. Die Längsseiten 7, 8 des Gehäuses 1 laufen zur Gehäusevorderseite 5 aufeinander flach zu. Damit hat das Gehäuse die Form einer Computer-Maus.

Das Gehäuse 2 kann beispielsweise als Spritzgießteil aus Kunststoff gefertigt sein. Grundsätzlich kann es aber auch aus anderen verträglichen Materialien, beispielsweise Metall oder Keramik bestehen. Es weist eine zentrale zylindrische Ausnehmung 9 auf, die an der Gehäuseunterseite 4 offen und an der Gehäuseoberseite 3 mit einer kreisförmigen durchstechbaren Membran 10 (Septum) verschlossen ist. Die in die Ausnehmung eingelegte Membran 10, die einen Durchmesser hat, der etwa dem Durchmesser der Ausnehmung entspricht, stützt sich an einem nicht dargestellten umlaufenden Anschlag an der Oberseite der Ausnehmung ab. Die Oberseite der Membran 10 liegt an der Gehäuseoberseite frei, so dass sie mit einer Injektionsnadel durchstochen werden kann.

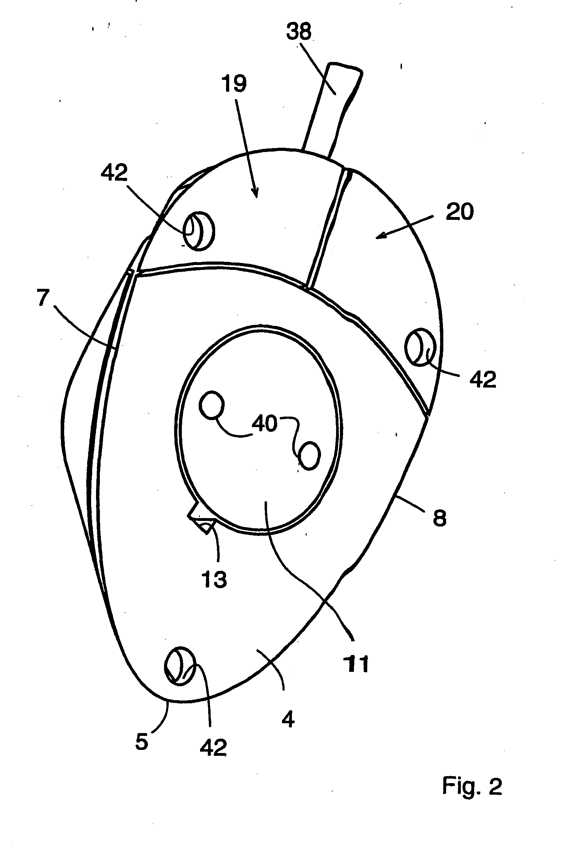

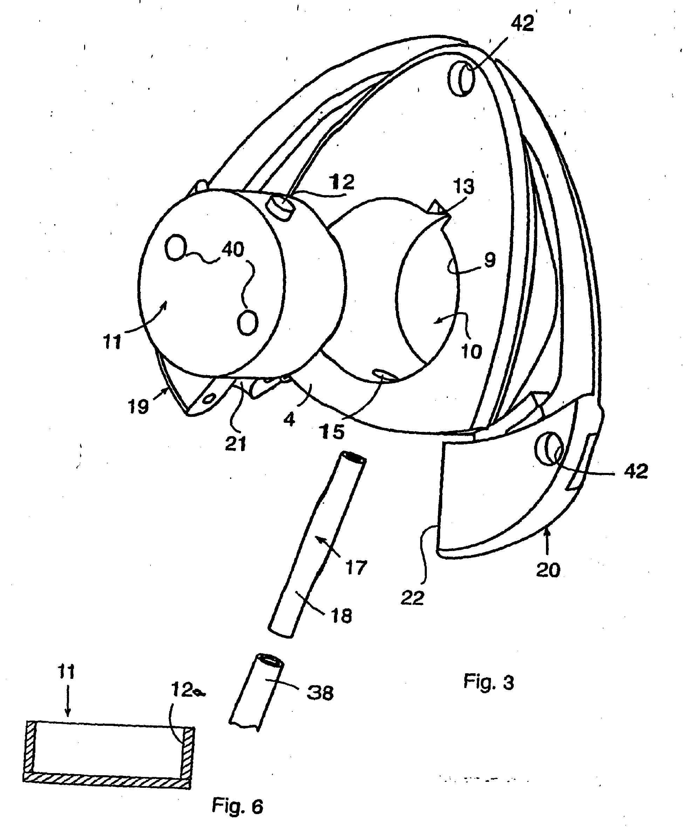

In die zentrale Gehäuseausnehmung 9 ist ein zylindrisches Einsatzstück 11 passend eingesetzt (Figur 2). In dem Einsatzstück 11 ist eine zylindrische Kammer 12a zur Aufnahme des zu applizierenden Wirkstoffs ausgebildet (Figur 6).

Von Vorteil ist, dass das Einsatzstück 11 unabhängig von dem Gehäuse 2 aus unterschiedlichen verträglichen Materialien, beispielsweise Kunststoff oder Metall, insbesondere Titan oder Keramik, gefertigt werden kann, ohne dass der gesamte Port verändert zu werden braucht. Dies kommt insbesondere dann zum Tragen, wenn das Einsatzstück beispielsweise mit über den Katheter aspirierten Blut in Kontakt kommen kann., da die unterschiedlichen Materialien bezüglich der Blutkqmpatibilität unterschiedliche Akzeptanz haben.

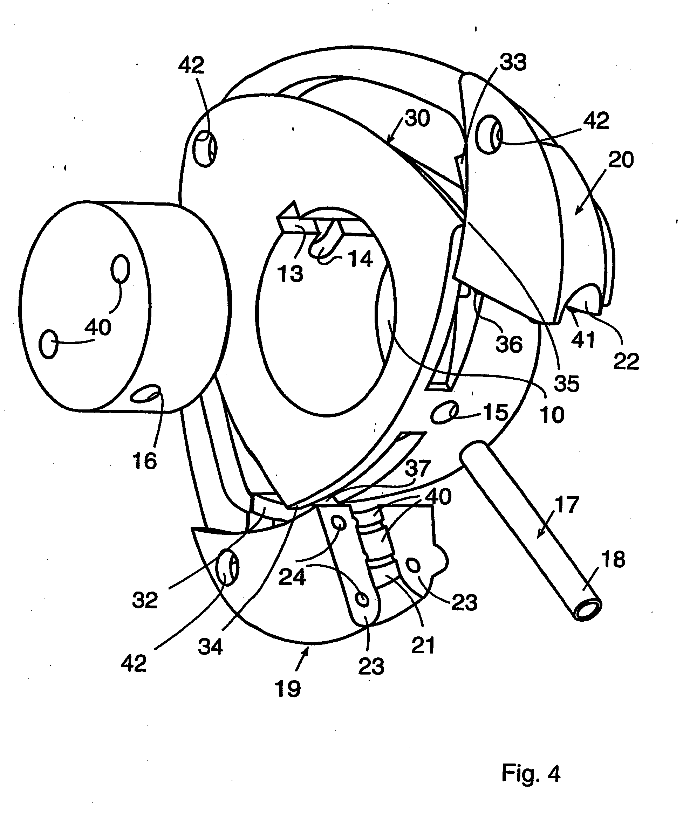

Das Einsatzstück 11 bildet zusammen mit dem Gehäuse 2 einen Bajonettverschluss. Hierzu weist das Einsatzstück 11 an der Außenseite einen vorspringenden zylindrischen Absatz 12 und die Ausnehmung 9 an der Wandung eine axiale Nut 13 auf, in die der Absatz 12 passend eingeschoben werden kann (Fig. 3). Zur Verriegelung des Absatzes 12 weist die Nut 13 eine seitliche Hinterschneidung 14 auf, in die nach Verdrehen des Einsatzstückes 11 der Absatz 12 greift (Fig. 4). Der Bajonettverschluss ist derart ausgelegt, dass das Einsatzstück 11 eine ausreichende Anpresskraft auf die Membran 10 ausübt, so dass sich die Membran 10 nach außen wölbt.

An der Rückseite 6 weist das Gehäuse 2 eine Bohrung 15 auf, die mit einer Bohrung 16 gleichen Durchmessers in der Zylinderwand des Einsatzstücks 11 fluchtet, wenn das Einsatzstück in das Gehäuse eingesetzt und durch Drehen verriegelt ist. Die Bohrungen 15 und 16 in dem Gehäuse und dem Einsatzstück 11 liegen diametral der Hinterschneidung 14 gegenüber, wobei die Nut 13 diesbezüglich seitlich versetzt ist. Aus dieser Anordnung ergeben sich für die Herstellung Vorteile bei der Entformung.

In den einander fluchtenden Bohrungen 15, 16 des Einsatzstücks 11 und des Gehäuses 2 sitzt eine rohrförmige Kanüle 17, deren vorstehendes Endstück das Anschlussstück 18 des Ports bildet, auf den das Endstück des Katheters 38 aufgeschoben wird. Vorzugsweise ist das Ende des Anschlussstücks 18 an der Außenseite konisch. Die Kanüle 17, die sich durch beide Bohrungen 15, 16 erstreckt, schafft nicht nur eine Fluidverbindung zwischen der Kammer 12a und dem Anschlussstück 18, sondern sichert gleichsam das Einsatzstück gegen Verdrehen. Dies hat folgende Vorteile bei der Montage.

Zur Montage wird das Einsatzstück 11 in die Gehäuseausnehmung 9 eingesetzt und verriegelt. Dies kann mit einem entsprechenden Werkzeug erfolgen, das in Bohrungen 40 des Einsatzstücks 11 greifende Vorsprünge aufweist. Anschließend wird die Kanüle 17 in die Bohrungen 15, 16 vorgeschoben. Nunmehr wird Klebstoff in die Nut 13 eingepresst, der sich über nicht dargestellte umlaufende Kanäle, die an der Wandung der Ausnehmung 9 des Gehäuses vorgesehen sind, gleichmäßig verteilt, so dass der Klebstoff die Spalte zwischen Einsatzstück 11, Membran 10, Kanüle 17 und Gehäuse 1 vollständig ausfüllt. Damit ist das Einsatzstück, die Membran und die Kanüle gegenüber dem Gehäuse abgedichtet. Zusätzliche Klemmvorrichtungen und dergleichen sind während des Aushärtens des Klebstoffes nicht erforderlich, da das in die Ausnehmung 9 eingesetzte Einsatzstück mittels des Bajonettverschlusses und der Kanüle 7 fixiert ist.

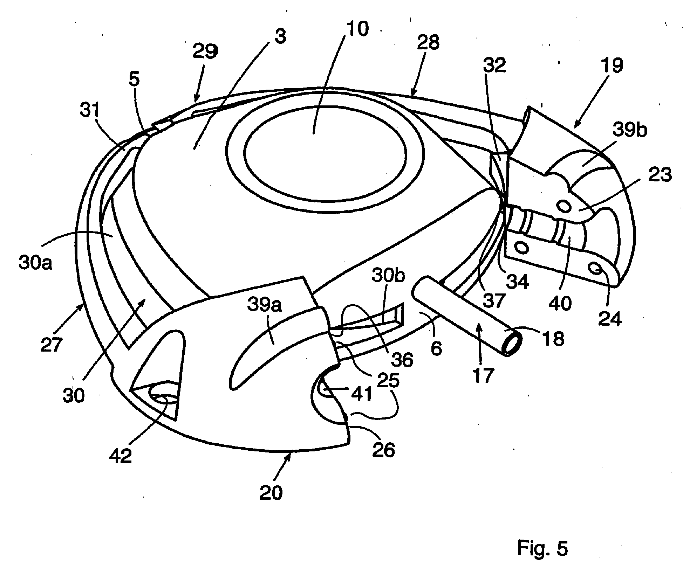

Zum klemmenden Fixieren des auf das Anschlussstück 18 aufgeschobenen Katheters 38 weist der Port zwei Klemmbacken 19, 20 auf, die jeweils eine dem Durchmesser der Kanüle entsprechende Klemmfläche 21, 22 haben. Die beiden Klemmbacken 19, 20 sind zwischen einer ersten Position, die in den Figuren 3 bis 5 dargestellt ist, und einer zweiten Position (Figuren 1 und 2) beweglich. In der zweiten Position umschließen die Klemmbacken 19, 20 den Katheter 38 fest, so dass der Katheter sicher auf dem Anschlussstück 18 sitzt. In der ersten Position hingegen stehen die Klemmbacken 19, 20 seitlich von dem Gehäuse ab, so dass zum einen ein ausreichender Freiraum zum Aufschieben des Katheters auf das Anschlussstück gegeben ist, und zum anderen eine Sichtkontrolle des auf das Anschlussstück aufgeschobenen Katheters möglich ist.

An dem Gehäuse 1 sind die Klemmbacken 19, 20 jeweils mit einem federnd ausgebildeten Befestigungsarm 27, 28 befestigt. Die Befestigungsarme 27, 28 bilden eine U-förmige Spange 29, die in an der Vorderseite 5 und den Längsseiten 7, 8 des Gehäuses 1 verlaufenden Führungsnuten 30 sitzt. Die Spange 29 ist nur mit ihrem bogenförmigen Mittelstück 31 an der Gehäusevorderseite 5 befestigt, so dass die Seitenstücke der Spange nach außen abspreizbar sind.

An den Innenseiten weisen die Klemmbacken 19, 20 jeweils einen Rasthaken 32, 33 auf, die beim Abspreizen der Befestigungsarme 27, 28 in den Führungsnuten 30 gleiten. Die Führungsnuten 30 weisen jeweils zwei Teilstücke auf, von denen sich das eine Teilstück 30a entlang der Gehäuselängsseite 7, 8 und das andere Teilstück 30b entlang der Gehäuserückseite 6 erstreckt. Im Übergangsbereich zwischen den beiden Teilstücken 30a und 30b bilden die Führungsnuten 30 vorspringende Absätze 34, 35. In der ersten Position, in der die Klemmbacken 19, 20 seitlich abstehen, stützen sich die Innenflächen der Rasthaken 32, 33 an den Absätzen 34, 35 ab.

In den Abschnitten 30b der Führungsnuten 30 an der Gehäuserückseite 6 sind jeweils Absätze 36, 37 ausgebildet, an denen sich die Rasthaken 32, 33 abstützen, wenn sich die Klemmbacken 19, 20 in der zweiten Position befinden, in der sie den Katheter klemmend fixieren. An der Stirnfläche 23 der einen Klemmbacke 19 sind Bohrungen 24 vorgesehen, in die Zapfen 25 greifen, die an der Stirnfläche 26 der anderen Klemmbacke 20 vorgesehen sind. Ferner sind an den Klemmflächen 21, 22 Klemmrippen 40, 41 vorgesehen.

Die Kontur der Spange 30 und die Konturen der Klemmbacken 19, 20 mit den Rasthaken 32, 33 entsprechen den Konturen der Führungsnuten 30 und der Kontur des Gehäuses, so dass sich die Spange und die Klemmbacken und das Gehäuse passend zusammenfügen. An der Gehäusevorderseite 5 und den Klemmbacken . 20, 21 sind Gewebefixierlöcher vorgesehen, um das Portsystem vernähen zu können.

Zwischen der Membran 10 und dem Anschlussstück 18 ist auf der Oberseite der Klemmbacke 19, 20 ein vorspringender Absatz 39 vorgesehen, der verhindert, das eine auf das Gehäuse auftreffende Injektionsnadel von dem Gehäuse abrutscht und den Katheter verletzt. Der vorspringende Absatz 39 besteht aus zwei Teilstücken 39a, 39b, von denen sich jeweils ein Teilstück über die Oberseite der entsprechenden Klemmbacke im wesentlichen senkrecht zur Längsachse des Anschlussstücks 18 erstreckt.

Trifft die Injektionsnadel zwischen der Membran und dem Anschlussstück 18 auf der Oberseite des Gehäuses 2 des Ports 1 auf, so könnte die Nadel in Richtung des Anschlussstücks 18 abrutschen und den Katheterschlauch perforieren. Dies würde den aufwendigen chirurgischen Austausch des Portsystems erforderlich machen. Falls die Perforation nicht erkannt wird, besteht zudem die Gefahr, dass der Wirkstoff nicht oder nur unzureichend an den Wirkort gelangt. Zusätzlich kann der Wirkstoff am falschen Wirkort, d.h. an der Perforation austreten. Der vorspringende Absatz 39 kann dies dadurch verhindern, dass die mit einer gewissen Kraft auf das Gehäuse auftreffende Injektionsnadel im Falle des in Richtung des Anschlussstücks 18 erfolgenden Abgleitens durch den Absatz 39 im wesentlichen senkrecht zum Anschlussstück seitlich weggeleitet wird.

## Patentansprüche

1. Port für einen Katheter mit
einer in einem Gehäuse (2) angeordneten Kammer (12) zur Aufnahme eines Wirkstoffes, die von einer durchstechbaren Membran (10) verschlossen ist,
einem mit der Kammer in Fluidverbindung stehenden Anschlussstück (18) zum Anschluss des Katheters, und
Mitteln zur Fixierung des Katheters an dem Anschlussstück
**dadurch gekennzeichnet, dass**
die Mittel zur Fixierung des Katheters an dem Anschlussstück zwei mit dem Gehäuse (2) verbundene Klemmbacken (19,20) mit einander gegenüberliegenden Klemmflächen (23, 24) aufweisen, wobei die Klemmbacken aus einer ersten Position, in der die Klemmbacken von dem Gehäuse seitlich abstehen, in eine zweite Position bewegbar sind, in der die Klemmbacken den Katheter zwischen deren Klemmflächen klemmend fixieren.

2. Port nach Anspruch '!, **dadurch gekennzeichnet, dass** die Klemmbacken (19, 20) mit federnd ausgebildeten Befestigungsarmen (27, 28) an dem Gehäuse (2) befestigt sind.

3. Port nach Anspruch 2, **dadurch gekennzeichnet, dass** die Befestigungsarme (27, 28) eine das Gehäuse (2) seitlich umfassende Spange (29) bilden, die an der dem Anschlussstück (18) gegenüberliegenden Seite an dem Gehäuse befestigt sind.

4. Port nach einem der Ansprüche 1 bis 3, **dadurch gekennzeichnet, dass** die Klemmbacken (19,20) in der zweiten Position einrastend an dem Gehäuse (2) festgelegt sind.

5. Port nach einem der Ansprüche 1 bis 4, **dadurch gekennzeichnet, dass** das Gehäuse Führungsnuten (30) aufweist, in denen die Klemmbacken (19, 20) geführt sind.

6. Port nach Anspruch 5, **dadurch gekennzeichnet, dass** in den Führungsnuten (30) Absätze (36,37) ausgebildet sind und die Klemmbacken (19, 20) Rasthaken (32, 33) aufweisen, die in der zweiten Position an den Absätzen einrastend festgelegt sind.

7. Port nach einem der Ansprüche 1 bis 6, **dadurch gekennzeichnet, dass** die Klemmbacken (19, 20) einander zugeordnete Zapfen (25) und Bohrungen (24) aufweisen, die in der zweiten Position ineinandergreifen.

8. Port nach einem der Ansprüche 1 bis 7, **dadurch gekennzeichnet, dass** die Kammer (12a) in einem Einsatzstück (11) ausgebildet ist, das in eine Ausnehmung (9) des Gehäuses (2) unter klemmender Zwischenlage der Membran (10) derart arretiert ist, dass das Einsatzstück einen Anpressdruck auf die Membran ausübt,

9. Port nach Anspruch 8, **dadurch gekennzeichnet, dass** das Einsatzstück (11) und das Gehäuse (2) als Bajonettverschluss ausgebildet sind.

10. Port nach einem Anspruch 8 oder 9, **dadurch gekennzeichnet, dass** das Einsatzstück (11) einen vorspringenden Absatz (12) und die Gehäuseausnehmung (9) eine Nut (13) mit einer seitlichen Hinterschneidung (14) aufweist, in der der vorspringende Absatz des Einsatzstücks sitzt

11. Port nach einem der Ansprüche 8 bis 10, **dadurch gekennzeichnet, dass** in dem Gehäuse (2) und dem Einsatzstück (11) miteinander fluchtende Bohrungen (15, 16) vorgesehen sind, und das Anschlussstück (18) eine in die Bohrungen des Gehäuses und Einsatzstücks eingesetzte Kanüle (17) ist.

12. Port nach Anspruch 10 oder 11, **dadurch gekennzeichnet, dass** die Hinterschneidung (14) und die miteinander fluchtenden Bohrungen (15,16) einander diametral gegenüberliegend angeordnet sind.

13. Port nach einem der Ansprüche 8 bis 12, **dadurch gekennzeichnet, dass** die Membran (10) und/oder das Einsatzstück (11) und/oder das Anschlussstück (18) mit dem Gehäuse (2) verklebt sind.

14. Port nach einem der Ansprüche 1 bis 13, **dadurch gekennzeichnet, dass** der Port ein Spritzgussteil ist.

## Claims

1. A port for a catheter with
a chamber (12) disposed in a housing (2) for accommodating an active substance, said chamber being sealed by a pierceable membrane (10),
a connecting piece (18) for the connection of the catheter, said connecting piece being in a fluid connection with the chamber, and
means for fixing the catheter to the connecting piece,
**characterised in that**
the means for fixing the catheter to the connecting piece comprise two clamping jaws (19, 20) connected to the housing (2) with clamping faces (23, 24) lying opposite one another, the clamping jaws being movable from a first position, in which the clamping jaws project laterally from the housing, into a second position, in which the clamping jaws fix the catheter in a clamped manner between the clamping faces.

2. The port according to claim 1, **characterised in that** the clamping jaws (19, 20) are fastened to the housing (2) with fastening arms (27, 28) designed in a spring-mounted manner.

3. The port according to claim 2, **characterised in that** the fastening arms (27, 28) form a clasp (29) surrounding the housing (2) laterally, said clasp being fastened to the housing at the side lying opposite the connecting piece (18).

4. The port according to any one of claims 1 to 3, **characterised in that** the clamping jaws (19, 20) in the second position are fastened in a snap-in manner to the housing (2)

5. The port according to any one of claims 1 to 4, **characterised in that** the housing comprises guide grooves (30), in which the clamping jaws (19, 20) are guided.

6. The port according claim 5, **characterised in that** shoulders (36, 37) are formed in the guide grooves (30) and the clamping jaws (19, 20) have snap-in hooks (32, 33), which in the second position are fixed in a snap-in manner at the shoulders.

7. The port according to any one of claims 1 to 6, **characterised in that** the clamping jaws (19, 20) comprise pegs (25) and holes (24) assigned to one another, which in the second position engage into one another.

8. The port according to any one of claims 1 to 7, **characterised in that** the chamber (12a) is formed in an insert (11), which is locked into a recess (9) of the housing (2) with the clamped interposition of the membrane (10), in such a way that the insert exerts a contact pressure on the membrane.

9. The port according to claim 8, **characterised in that** the insert (11) and the housing (2) are designed as a bayonet lock.

10. The port according to claim 8 or 9, **characterised in that** the insert (11) comprises a projecting shoulder (12) and the housing recess (9) a groove (13) with a lateral undercut (14) in which the projecting shoulder of the insert sits.

11. The port according to any one of claims 8 to 10, **characterised in that** holes (15, 16) aligned with one another are provided in the housing (2) and the insert (11), and the connecting piece (18) is a cannula (17) inserted into the holes of the housing and insert.

12. The port according to claim 10 or 11, **characterised in that** the undercut (14) and the holes (15, 16) aligned with one another are disposed diametrically opposite one another.

13. The port according to any one of claims 8 to 12, **characterised in that** the membrane (10) and/or the insert (11) and/or the connecting piece (18) are glued to the housing (2).

14. The port according to any one of claims 1 to 13, **characterised in that** the port is an injection-moulded part.

## Revendications

1. Système d'accès pour un cathéter comportant
◆ une chambre (12), disposée dans un boîtier (2), destinée à contenir un produit actif, chambre qui est fermée par une membrane (10) pouvant être percée, une pièce de raccordement (18), en liaison par fluide avec la chambre, destinée à raccorder le cathéter et
◆ des moyens de fixation du cathéter à la pièce de raccordement,
**caractérisé en ce que** les moyens de fixation,du cathéter à la pièce de raccordement comportent deux mâchoires de serrage (19, 20), reliées au boîtier (2), avec des surfaces de serrage opposées (23, 24), sachant que les mâchoires de serrage peuvent être déplacées d'une première position, dans laquelle les mâchoires de serrage sont éloignées latéralement du boîtier, à une deuxième position dans laquelle les mâchoires de serrage fixent le cathéter par serrage entre leurs surfaces de serrage.

2. Système d'accès selon la revendication 1, **caractérisé en ce que** les mâchoires de serrage (19, 20) sont fixées au boîtier (2) au moyen de branches de fixation (27, 28) flexibles.

3. Système d'accès selon la revendication 2, **caractérisé en ce que** les branches de fixation (27, 28) forment une boucle fermoir (29) entourant latéralement le boîtier (2), branches qui sont fixées au boîtier sur le côté opposé à la pièce de raccordement (18) :

4. Système d'accès selon l'une quelconque des revendications 1 à 3, **caractérisé en ce que** les mâchoires de serrage (19, 20) sont fixées au boîtier (2) par encliquetage dans la deuxième position.

5. Système d'accès selon l'une quelconque des revendications 1 à 4, **caractérisé en ce que** le boîtier comporte des rainures de guidage (30) dans lesquelles sont guidées les mâchoires de serrage (19, 20).

6. Système d'accès selon la revendication 5, **caractérisé en ce que** des épaulements (36, 37) sont réalisés dans les rainures de guidage (30) et les mâchoires de serrage (19, 20),comportent des crochets à cliquet (32, 33) qui sont fixés sur les épaulements par enclenchement dans la deuxième position.

7. Système d'accès selon l'une quelconque des revendications 1 à 6, **caractérisé en ce que** les mâchoires de serrage (19, 20) comportent des tourillons (25) et des trous (24) attribués les uns aux autres qui s'engrènent les uns dans les autres dans la deuxième position.

8. Système d'accès selon l'une quelconque des revendications 1 à 7, **caractérisé en ce que** la chambre (12a) est réalisée dans une pièce à insérer (11) qui est bloquée dans un évidement (9) du boîtier (2) par l'insertion de la membrane (10), ayant une action bloquante, de sorte que la pièce à insérer exerce une pression de serrage sur la membrane.

9. Système d'accès selon la revendication 8, **caractérisé en ce que** la pièce à insérer (11) et le boîtier (2) sont réalisés avec un verrouillage à baïonnette.

10. Système d'accès selon la revendication 8 ou 9, **caractérisé en ce que** la pièce à insérer (11) comporte un épaulement en saillie (12) et l'évidement de boîtier (9) une rainure (13) avec une contre-dépouille latérale (14) dans laquelle se trouve l'épaulement en saillie de la pièce à insérer.

11. Système d'accès selon l'une quelconque des revendications 8 à 10, **caractérisé en ce qu'**on a prévu des trous (15, 16) en alignement les uns avec les autres dans le boîtier (2) et la pièce à insérer (11), et la pièce de raccordement (18) est une canule (17) insérée dans les trous du boîtier et de la pièce à insérer.

12. Système d'accès selon la revendication 10 ou 11, **caractérisé en ce que** la contre-dépouille (14) et les trous (15, 16) en alignement les uns avec les autres sont disposés face à face dans le sens diamétral.

13. Système d'accès selon l'une quelconque des revendications 8 à 12, **caractérisé en ce que** la membrane (10) et/ou la pièce à insérer (11) et/ou la pièce de raccordement (18) sont collées au boîtier (2).

14. Système d'accès selon l'une quelconque des revendications 1 à 13, **caractérisé en ce que** le système d'accès est une pièce moulée par injection.
